# EUROPEAN PATENT APPLICATION

(11) **EP 1 693 375 A1**
(43) Date of publication of application: **23.08.2006**
(21) Application number: 05003654.0
(22) Date of filing: 21.02.2005
(51) Int. Cl.: C07D 495/04

(54) **Process for preparing clopidrogel hydrogen sulfate of form I**

(71) Applicant: KRKA, tovarna zdravil, d.d., Novo mesto, 8501 Novo mesto (SI)
(72) Inventor: Ruzic, Milos, SLO-3000 Celje (SI); Kotar-Jordan, B., SLO-8311 Kostanjevica na Krki (SI); Smrkolj, Matej, SLO-1411 Izlake (SI); Gerksic, Samo, SLO-1000 Ljubljana (SI); Vrancic, Damir, SLO-8000 Novo mesto (SI); Benedik, Milena, SLO-8321 Brusnice (SI); Gricar, Mira, SLO-8210 Trebnje (SI)
(74) Representative: UEXKÜLL & STOLBERG

(57) **Abstract**

The invention relates to a process for the preparation of form I clopidogrel hydrogen sulfate through suspending clopidogrel hydrogen sulfate in an alkane.

## Description

### Technical field

The invention relates to a process for preparing the hydrogen sulfate of clopidogrel of form I.

Clopidogrel is the generic name for methyl (+)-(S)-α-(2-chlorophenyl)-6,7-dihydrothieno[3,2-c]pyridine-5(4*H*)-acetate and is a compound which has shown to have activity with inhibitory properties towards platelet aggregation, it interferes with the mechanism of formation of arterial and venous thromboses and is useful for the treatment and prevention of platelet disorders.

### Technical problem

According to prior art processes, the preparation as well as the crystallization of the hydrogen sulfate of clopidogrel in particular of the presently known form I hydrogen sulfate of clopidogrel, usually results in a mixture of different polymorphic forms. In particular the preparation of pure clopidogrel hydrogen sulfate of Form I which is substantially free of clopidogrel hydrogen sulfate of Form II is difficult to achieve.

In view of the above disadvantages of the prior art processes, there is a need for a process which leads to the form I hydrogen sulfate of clopidogrel having a high polymorphic purity and a high crystallinity.

The invention provides such a process.

### Background of the invention

EP 0 099 802 discloses clopidogrel as well as the platelet anti-aggregating activity of this compound.

EP 0 281 459 describes the existence of two enantiomers of clopidogrel and the crystalline hydrogen sulfate salts thereof with the dextrorotatory isomer having substantially higher therapeutic activity. A process for producing crystalline hydrogen sulfate of clopidogrel involves crystallization from cold acetone.

WO 99/65915 describes two polymorphic forms of clopidogrel hydrogen sulfate, designated form I and form II. Both the form I and the form II hydrogen sulfate are characterized by e.g. X-ray data, FT-IR and DSC. The preparation of the form I and form II clopidogrel hydrogen sulfate is effected by dissolving clopidogrel base in acetone with addition of sulphuric acid.

The mother liquor of the reaction mixture is said to release form II clopidogrel hydrogen sulfate after 3 to 6 months. The aquecus-acetone mother liquors contain 0.3 to 1 % of water and about 10% of clopidogrel.

WO 03/051362 discloses crystalline forms III, IV, V and VI of clopidogrel hydrogen sulfate and the amorphous form of clopidogrel hydrogen sulfate, as well as pharmaceutical compositions thereof and processes for preparation of these compounds. Clopidogrel hydrogen sulfate Form III is said to be a solvate of 1-butanol, Form IV of isopropanol, Form V of 2-butanol and Form VI of 1-propanol. The document is, however, silent on the usefulness of alkanes to prepare form I clopidogrel hydrogen sulfate.

WO 2004/081015 and WO 2005/003138 describe an amorphous form of clopidogrel hydrogen sulfate and processes for its preparation.

Finally, WO 2004/081016 discloses various polymorphic and amorphous forms of clopidogrel hydrogen sulfate. It also discloses processes for preparing the form I, but these processes employ acetone rather than alkanes.

### Detailed description of the invention

According to the present invention a process for the preparation of form I of clopidogrel hydrogen sulfate is provided which comprises
(a) forming a suspension of amorphous clopidogrel hydrogen sulfate in a liquid medium comprising at least one C₆ to C₁₀ alkane,
(b) agitating the suspension for at least 40 hours at a temperature of at least 30°C, and
(c) separating the formed clopidogrel hydrogen sulfate of form I.

It has surprisingly been found out that such a simple procedure allows an easy and very economical access to the highly desired form I of clopidogrel hydrogen sulfate.

Clopidogrel hydrogen sulfate of form I refers to the crystalline form of clopidogrel hydrogen sulfate as disclosed in WO 99/65915. It shows in the powder X-ray diffractogram the following characteristic peaks expressed as interplanar distances at approximately 9.60; 3.49; 3.83; 3.80; 4.31; 8.13; 4..80; 3.86; 5.80 and 4.95 x 10⁻¹⁰m and in the IR spectrum characteristic absorptions at 2987, 1753, 1222, 1175 and 841 cm¹.

The forming of a suspension in step (a) can be accomplished in several ways.

Firstly, clapidogrel hydrogen sulfate of amorphous form can be suspended in the selected liquid medium.

Secondly, the suspension may in-situ be formed. In a preferred embodiment of the invention this is achieved by reacting clopidogrel base with sulphuric acid in the selected liquid medium to form the desired suspension of clopidogrel hydrogen sulfate. This offers the advantage that for both the preparation of the hydrogen sulfate as well as the preparation of the form I of clopidogrel hydrogen sulfate the same liquid medium can be used.

The liquid medium in step (a) of the process according to the invention is preferably comprising the C₆ to C₁₀ alkane or mixtures thereof as only organic solvent. It is further preferred that the liquid medium consists of the C₆ to C₁₀ alkane or mixtures thereof.

The C₆ to C₁₀ alkane is preferably heptane, in particular n-heptane. It has been shown that amorphous clopidogrel hydrogen sulfate treated in the process of the invention with heptane shows a surprisingly high conversion into form I even though conventional treatments with other solvents tend to favour conversion into e.g. form II material.

In step (b), the suspension is agitated for at least 40 hours at a temperature of at least 30°C. It is preferred that the suspension is agitated for 40 to 65 hours. It also preferred that the suspension is agitated at a temperature of 30 to 55°C.

The agitating can for example be achieved by mixing or stirring.

It has surprisingly been found out that this step (b) results in conversion of the starting material to form I clopridogrel hydrogen sulfate. It is assumed that in particular the specific period of time and temperature for the agitating ensures a high conversion.

In step (c) the clopidogrel hydrogen sulfate of form I is separated from the suspension and this can for example be achieved be filtration or centrifuging.

In a preferred embodiment of the invention, the process also includes the further step (d) wherein the separated clopidogrel hydrogen sulfate of form I is allowed to stand for at least 10 days to enhance its crystallinity. Such an additional treatment is for example desirable if the separated form I material includes significant amounts of amorphous material. This may be present due to an incomplete conversion of amorphous clopidogrel hydrogen sulfate which has been used as starting material in step (a). It is further preferred that the separated clopidogrel hydrogen sulfate obtained in step (c) has not been further treated before subjecting it to this step (d). It is preferred that the separated form I material is allowed to stand for 10 to 90 days. It is further preferred that the temperature upon standing is kept in the region of 0 to 55°C and preferably 20 to 40°C.

Further, it has proved advantageous if the product left for standing comprises less than 5% by weight of residual solvents. Such a degree of solvents can be adjusted by drying in a drier, like a vacuum drier.

It is assumed that step (d) is capable of effecting a solid phase crystallization of form I material thereby resulting in an improved conversion of the amorphous material into form I material. It may be possible to enhance this solid phase crystallization by UV-light and/or an elevated temperature.

Generally, the form I hydrogen sulfate of clopidogrel is rather difficult to be prepared in substantially pure form, because the formation of the thermodynamically more stable form II is favoured. However, by using the above process according to the invention it was possible to prepare form I hydrogen sulfate of clopidogrel not only in high yields, but also with a high polymorphic purity and crystallinity. Thus, the process according to the present invention allows the producing of form I hydrogen sulfate of clopidogrel substantially free from form II and solvates.

The invention is in the following further illustrated by examples.

### Examples

### Example 1

5.00 g of amorphous clopidogrel hydrogen sulfate were added to a reaction flask under an inert atmosphere. Subsequently, 15 ml of n-heptane were added to give a suspension, and the suspension was agitated for 50 hours at a temperature of 30°C.

The product was separated by filtration under an inert atmosphere and the obtained wet product was dried for 2 hours at 50 °C and overnight at room temperature in a vacuum drier.

### Example 2

The procedure as in example 1 was repeated with the exception that 45 ml n-heptane were used.

### Example 3

The procedure as in example 1 was repeated with the exception that the suspension was agitated at 40°C.

### Example 4

The procedure as in example 2 was repeated with the exception that the suspension was agitated at 40°C.

### Example 5

The procedure as in example 3 was repeated with the exception that 2.5 g of amorphous clopidogrel hydrogen sulfate and 13.5 ml n-heptane were used.

### Example 6

The procedure as in example 5 was repeated with the exception that the suspension was agitated at 50°C.

### Example 7

The procedure as in example 5 was repeated with the exception that 22.5 ml n-heptane were used.

### Example 8

The procedure as in example 7 was repeated with the exception that the suspension was agitated at 50°C.

### Example 9

The procedure as in example 1 was repeated with the exception that 100 g of amorphous clopidogrel hydrogen sulfate, 900 ml n-heptane were used, the suspension was agitated for 44 hours at 40°C and the wet product was dried for 4 hours at 50 °C and overnight at room temperature in a vacuum drier.

### Example 10

The procedure as in example 1 was repeated with the exception that 40 g of amorphous clopidogrel hydrogen sulfate, 360 ml n-heptane were used, and the suspension was agitated for 44 hours at 40°C and the wet product was dried for 3 hours at 50 °C and overnight at room temperature in a vacuum drier.

To enhance the crystallinity of the products obtained by any one of the examples 1 to 10, they were adjusted to a content of residual solvents of less than 5 % by weight and then packed in a colored vial and left at room temperature for a sufficient period of time to achieve further crystallization of remaining amorphous material. After the end of such a further solid phase crystallization remaining solvents can be removed in a vacuum drier.

By using X-ray and NIR analyses the degree of the conversion and the end of the crystallization process was determined.

The above experiments show that the process according to the invention allows certain variations without affecting the production of the form I of clopidogrel hydrogen sulfate.

### Example 11

100 g of a solution of clopidogel in isopropanol (which contained 22 to 27 % clopidogrel) was heated under inert atmosphere. The solvent was distilled off at reduced pressure and an oily residue was obtained. Subsequently, heptane was added two or three times (2 or 3 x 100 ml) and each time the solvent was distilled off again to finally give 26.5 g of oily residue. 120 ml of heptane were added to this residue and the mixture was heated to 40°C under inert atmosphere. A mixture of sulphuric acid (molar ratio relative to clopidogel was 0.9 to 1.3) and 120 ml heptane was added under inert atmosphere. The obtained mixture was agitated with_a mechanically stirrer for 3 to 4 days at 40°C in such a way that the lump of solids stayed untouched at the bottom of flask for some time. After 4 to 8 hours, the lump was flaking off and after about 1 day a suspension of clopidogel hydrogen sulfate was formed. After the predetermined time the product was separated by filtration under an inert atmosphere and dried in vacuum for 2 hours at 50°C. 23.5 to 27.6 g of product having a content of residual solvent below 5 % by weight were obtained.

This product was then subjected to a solid phase crystallization as described in example 10 and the crystallization process was followed by the mentioned analytical techniques.

After finishing the crystallization a highly pure clopidogrel hydrogen sulfate of form I was obtained.

## Claims

1. Process for preparing clopidogrel hydrogen sulfate of form I, which comprises
(a) forming a suspension of amorphous clopidogrel hydrogen sulfate in a liquid medium comprising at least one C₆ to C₁₀ alkane,
(b) agitating the suspension for at least 40 hours at a temperature of at least 30°C, and
(c) separating the formed clopidogrel hydrogen sulfate of form I.

2. Process according to claim 1, wherein the C₆ to C₁₀ alkane is heptane, in particular n-heptane.

3. Process according to claim 1 or 2, wherein the liquid medium comprises the at least one C₆ to C₁₀ alkane as only organic solvent.

4. Process according to any one of claims 1 to 3, wherein the suspension is agitated for 40 to 65 hours.

5. Process according to any one of claims 1 to 4, wherein the suspension is agitated at a temperature of 30 to 55°C.

6. Process according to any one of claims 1 to 5, which further comprises the step
(d) allowing the separated clopidogrel hydrogen sulfate of form I to stand for at least 10 days to enhance its crystallinity.

7. Process according to claim 6, wherein the separated clopidogrel hydrogen sulfate used in step (d) has a content of residual solvents of less than 5 % by weight.

8. Process according to any one of claims 1 to 7, wherein the suspension of step (a) is formed by reacting clopidogrel with sulphuric acid in the liquid medium.
